(19) Europäisches Patentamt
European Patent Office
Office européen des brevets

(11) Veröffentlichungsnummer: **0 011 756**
**A2**

(12) # EUROPÄISCHE PATENTANMELDUNG

(21) Anmeldenummer: **79104437.3**

(22) Anmeldetag: **12.11.79**

(51) Int. Cl.³: **B 41 M 5/12**

(30) Priorität: **22.11.78 DE 2850533**

(43) Veröffentlichungstag der Anmeldung:
**11.06.80 Patentblatt 80/12**

(84) Benannte Vertragsstaaten:
**BE CH DE FR GB IT**

(71) Anmelder: **BASF Aktiengesellschaft**
**Carl-Bosch-Strasse 38**
**D-6700 Ludwigshafen(DE)**

(72) Erfinder: **Oberlinner, Andreas, Dr. Dipl.-Chem.**
**Bruesseler Ring 53**
**D-6700 Ludwigshafen(DE)**

(54) **Druckempfindliches Aufzeichnungsmaterial.**

(57) Druckempfindliches Aufzeichnungsmaterial, das als Farbbildner Verbindungen der Formel

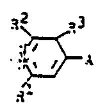

(I)

enthält, in der
$R^1$ und $R^2$ Wasserstoff, $C_1$-bis $C_4$-Alkyl, oder gegebenenfalls durch $C_1$-bis $C_4$-Alkyl, Methoxy, Äthoxy, Chlor oder Brom substituiertes Phenyl, $R^3$ Wasserstoff oder $R^3$ zusammen mit $R^2$ einen gesättigten carbocyclischen Fünf- oder Sechsring und A einen Rest der Formel

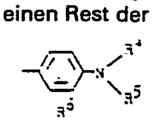 , 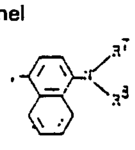 oder 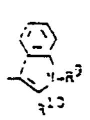

bedeuten, worin $R^4$ und $R^5$
für Wasserstoff, gegebenenfalls durch Chlor substituiertes $C_1$- bis $C_{12}$-Alkyl, Cyanäthyl, $C_7$- bis $C_{10}$-Phenalkyl oder

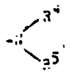 für Pyrrolidinyl, Piperidinyl, Morpholinyl, N'-$C_1$- bis $C_4$-Alkylpiperazinyl oder Isoindolinyl oder $R^4$ für Wasserstoff, $C_1$- bis $C_4$-Alkyl, 2-Cyanäthyl oder Benzyl und $R^5$ für gegebenenfalls durch Halogen, $C_1$- bis $C_4$-Alkyl oder $C_1$- bis $C_4$-Alkoxy substituiertes Phenyl, $R^6$ für Wasserstoff, $C_1$- bis $C_4$-Alkyl, $C_1$- bis $C_4$-Alkoxy, Chlor oder Brom, $R^7$ für Wasserstoff oder $C_1$- bis $C_4$-Alkyl, $R^8$ für $C_1$- bis $C_4$-Alkyl, gegebenenfalls $C_1$- bis $C_4$-Alkyl oder $C_1$ bis $C_4$-Alkoxy substituiertes Phenyl oder Cyclohexyl, $R^9$ für Wasserstoff, $C_1$- bis $C_{12}$-Alkyl oder $C_7$- bis $C_{10}$-Phenalkyl und $R^{10}$ für Wasserstoff, $C_1$- bis $C_{10}$-Alkyl oder für gegebenenfalls durch $C_1$- bis $C_4$-Alkyl oder $C_1$- bis $C_4$-Alkoxy substituiertes Phenyl stehen.

Die Diazepinderivate (I) sind gelbe bis farblose Verbindungen, die mit Elektronenacceptoren gelbe bis rote Färbungen geben. Die Farbbildner geben in Mischung mit Farbbildnern, die mit Elektronenacceptoren blaue oder grüne Farbtöne liefern, schwarze Färbungen.

**EP 0 011 756 A2**

Patentansprüche

1. Druckempfindliches Aufzeichnungsmaterial, dadurch gekennzeichnet, daß dieses als Farbbildner Diazepinderivate der allgemeinen Formel

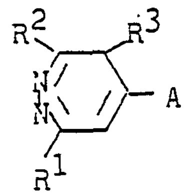 ,

enthält, in der
$R^1$ und $R^2$ unabhängig voneinander Wasserstoff, $C_1$- bis $C_4$-Alkyl, Phenyl oder durch $C_1$- bis $C_4$-Alkyl, Methoxy, Äthoxy, Chlor oder Brom substituiertes Phenyl,
$R^3$ Wasserstoff oder zusammen mit $R^2$ einen gesättigten carbocyclischen Fünf- oder Sechsring und
A einen Rest der Formel

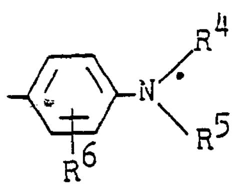 , 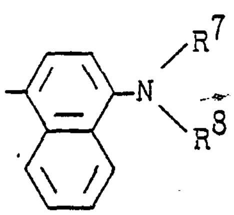 oder 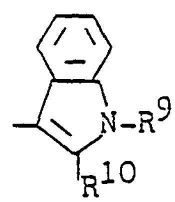

bedeuten, worin
$R^4$ und $R^5$ unabhängig voneinander für Wasserstoff, gegebenenfalls durch Chlor substituiertes $C_1$- bis $C_{12}$-Alkyl, Cyanäthyl, $C_7$- bis $C_{10}$-Phenalkyl oder die Gruppe

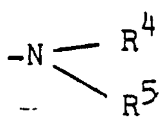 für Pyrrolidinyl, Piperidinyl, Morpholinyl, N'-$C_1$- bis $C_4$-Alkyl-piperazinyl oder Isoindolinyl, oder

BAD ORIGINAL

$R^4$ für Wasserstoff, $C_1$- bis $C_4$-Alkyl, 2-Cyanäthyl oder Benzyl und

$R^5$ für Phenyl oder durch Halogen, $C_1$- bis $C_4$-Alkyl oder $C_1$- bis $C_4$-Alkoxy substituiertes Phenyl,

$R^6$ für Wasserstoff, $C_1$- bis $C_4$-Alkyl, $C_1$- bis $C_4$-Alkoxy, Chlor oder Brom,

$R^7$ für Wasserstoff oder $C_1$- bis $C_4$-Alkyl,

$R^8$ für $C_1$- bis $C_4$-Alkyl, Phenyl, durch $C_1$- bis $C_4$-Alkyl oder $C_1$- bis $C_4$-Alkoxy substituiertes Phenyl oder Cyclohexyl,

$R^9$ für Wasserstoff, $C_1$- bis $C_{12}$-Alkyl oder $C_7$- bis $C_{10}$-Phenalkyl und

$R^{10}$ für Wasserstoff, $C_1$- bis $C_{10}$-Alkyl, Phenyl oder durch $C_1$- bis $C_4$-Alkyl oder $C_1$- bis $C_4$-Alkoxy substituiertes Phenyl stehen.

2. Druckempfindliches Aufzeichnungsmaterial gemäß Anspruch 1, dadurch gekennzeichnet, daß in der Formel $R^1$ und $R^2$ unabhängig voneinander für $C_1$- bis $C_4$-Alkyl, $R^3$ für Wasserstoff und A für den Rest der Formel

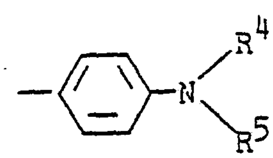

stehen, in der $R^4$ und $R^5$ die im Anspruch 1 angegebene Bedeutung haben.

3. Druckempfindliches Aufzeichnungsmaterial gemäß Anspruch 2, dadurch gekennzeichnet, daß $R^1$ und $R^2$ für tert.-Butyl, $R^3$ für Wasserstoff und $R^4$ und $R^5$ unabhängig voneinander für $C_1$- bis $C_4$-Alkyl oder Benzyl stehen.

iertes $C_1$- bis $C_{12}$-Alkyl wie Methyl, Äthyl, Propyl, n-Butyl, Pentyl, Hexyl, Octyl, Nonyl, Decyl und Dodecyl, 2-Chloräthyl, Chlorhexyl; $C_7$- bis $C_{10}$-Phenalkyl wie Benzyl, 2-Phenyläthyl, 2-Phenylpropyl, 3-Phenylpropyl, 4-Phenylbutyl.

$R^5$ kann auch für Phenyl oder durch Halogen, Alkyl oder Alkoxy substituiertes Phenyl wie 4-Chlorphenyl, 4-Bromphenyl, 4-Methoxyphenyl, 4-Äthoxyphenyl, 4-Methylphenyl, 4-Äthylphenyl, 4-Isopropylphenyl stehen. In diesem Falle muß $R^4$ Wasserstoff, $C_1$- bis $C_4$-Alkyl wie Methyl, Äthyl, Propyl oder Butyl, 2-Cyanäthyl oder Benzyl sein.

Für $R^6$ kommt $C_1$- bis $C_4$-Alkyl wie Methyl, Äthyl, n- oder i-Propyl, i-Butyl, sec.-Butyl und tert.-Butyl, $C_1$- bis $C_4$-Alkoxy wie Methoxy, Äthoxy, n- oder i-Propoxy und n- oder i-Butoxy oder vorzugsweise Wasserstoff in Betracht.

$R^7$ ist Wasserstoff oder $C_1$- bis $C_4$-Alkyl wie Methyl, Äthyl, Propyl oder n-Butyl.

$R^8$ steht für $C_1$- bis $C_4$-Alkyl z.B. Methyl, Äthyl, n- oder i-Propyl, n-Butyl, für Phenyl oder substituiertes Phenyl wie 4-Methylphenyl, 4-Äthylphenyl, 4-Methoxyphenyl, 4-Äthoxyphenyl oder Cyclohexyl.

Für $R^9$ sind außer Wasserstoff im einzelnen z.B. zu nennen: für $C_1$- bis $C_{12}$-Alkyl: Methyl, Äthyl, Propyl, Butyl, Pentyl, Hexyl, Octyl, Nonyl, Decyl und Dodecyl und $C_7$- bis $C_{10}$-Phenalkyl wie Benzyl, 2-Phenyläthyl, 3-Phenylpropyl, 2-Phenylpropyl und 4-Phenylbutyl.

Als Substituenten kommen für $R^{10}$ außer Wasserstoff und Phenyl als $C_1$- bis $C_{10}$-Alkyl und substituiertes Phenyl z.B. in Betracht: Methyl, Äthyl, Propyl, Butyl, Pentyl, Hexyl,

BAD ORIGINAL

Octyl, Decyl, 4-Methoxyphenyl, 4-Äthoxyphenyl, 4-Methylphenyl und 4-Äthylphenyl.

Die Diazepinderivate der Formel I mit $R^1$ und $R^2$ = Phenyl sind gelbe, die mit $R^1$ und $R^2$ = Alkyl sind farblose Verbindungen. Die erfindungsgemäß zu verwendenden Diazepine haben die Eigenschaft, daß sich ihre Absorption bathochrom um 150 bis 200 nm verschiebt, wenn sie in einem inerten organischen Lösungsmittel, vorteilhafterweise in gelöster Form, mit elektronenanziehenden Materialien in Kontakt gebracht werden, d.h. es entstehen gelbe bis rote Färbungen. Typische Beispiele für Elektronenacceptorsubstanzen sind Carbon- oder Mineralsäuren, Kaolin, Bentonit, aktivierter Ton, Aluminiumsilikat, Attapulgit oder jeder beliebige saure Ton, sauer reagierende polymere Materialien, wie Kondensationsprodukte aus Phenolen und/oder Phenolsulfonsäuren, ferner Metalloxide oder -salze wie Zinkoxid, Aluminiumoxid, Eisenstearat oder Cobaltnaphthenat.

Aufgrund dieser Eigenschaften sind die Verbindungen der Formel I als Farbbildner (Chromogene) in druckempfindlichen Aufzeichnungs- und Kopiermaterialien oder sonstigen Durchschreibesystemen geeignet.

Vorzugsweise enthalten die erfindungsgemäßen druckempfindlichen Aufzeichnungsmaterialien die Farbbilder in organischen hochsiedenden Lösungsmitteln wie Chlorparaffinen, halogenierten oder teilhydriertem Biphenyl, Alkylbenzol, Alkylnaphthalin, alkylierten Dibenzylbenzol, Paraffinöl, Mineralöl oder auch in einem üblichen Lösungsmittel wie Toluol, Xylol, in Form einer Lösung oder Suspension gegebenenfalls zusammen mit weiteren Farbbildnern, in Mikrokapseln eingeschlossen. Im Kontakt mit elektronenanziehenden Materialien entsteht bei der Zerstörung der Kapsel, z.B. beim Schreib- und Typendruck eine Durchschrift in gelben

bis roten Farbtönen. So erhält man mit einem Aufzeichnungsmaterial, das Diazepinderivate der Formel I mit $R^1$ und $R^2$ = Phenyl und $R^4$ und $R^5$ = Methyl enthält, auf sog. Nehmerpapier rote Durchschriften. Dagegen geben die Diazepinderivate der Formel I mit $R^1$ und R = Alkyl Durchschriften in intensiven Gelb- bis Orangefarbtönen.

Die 3,7-Dialkyldiazepinverbindungen der Formel I können vorteilhaft zusammen mit anderen Farbbildnern, die mit Elektronenakzeptorsubstanzen blaue oder grüne Farbtöne entwickeln, zur Herstellung schwarz durchschreibender Kopierpapiere verwendet werden.

Verfahren zur Herstellung von Mikrokapseln sind z.B. in den US-PS 2 800 457, 2 800 458 (Gelatinekapseln) und 3 872 023 (Polymerisatkapseln) beschrieben.

Zur Herstellung von mit Mikrokapseln beschichteten druckempfindlichen Aufzeichnungsmaterial wird die erhaltene wäßrige Mikrokapseldispersion nach der Zugabe von in der Papierverarbeitung üblichen Bindemitteln und gegebenenfalls von weiteren Zusätzen wie Abstandshaltern, z.B. Cellulosepulver oder -mehl, Schutzkolloiden oder die Viskosität der aufzutragenden Mikrokapseldispersion stabilisierenden Verbindungen, dann auf den Träger, z.B. Papier, aufgebracht und der beschichtete Träger getrocknet.

Die Mikrokapseldispersion kann auf den Träger mit Hilfe des Bürstenverfahrens, z.B. mit der Luftbürste oder mit Druckverfahren, z.B. mit Druckzylindern, auf denen viele tiefgeätzte Gravuren dicht nebeneinander angeordnet sind, aufgebracht werden.

Als Trägermaterial kommen Folien, vorzugsweise Papier, in Betracht. Dabei ist unter Papier nicht nur aus Cellulose-

BAD ORIGINAL

Fasern hergestelltes Papier, sondern auch Papier zu verstehen, in dem die Cellulosefasern teilweise bis vollständig durch synthetische Fasern aus Polymerisaten ersetzt sind.

Das erfindungsgemäße Aufzeichnungsmaterial kann die Farbbildner auch in feinverteilter Form in Wachs oder Öl-Wachsmischungen enthalten. Aufzeichnungsmaterial dieser Art kann z.B. nach dem in der US-PS 3 103 404 beschriebenen Verfahren durch Beschichten von Trägern wie Folien oder Papier hergestellt werden.

Man erhält druckempfindliche Materialien, die zum Durchschreiben auf mit Elektronenakzeptorsubstanzen beschichteten Papieren geeignet sind und die nach Gebrauch wie Kohlepapier entfernt werden.

Als Farbbildner sind für diesen Zweck Diazepine der Formel Ia von besonderer Bedeutung

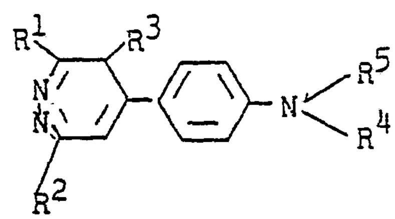

(Ia),

in der
$R^1$ und $R^2$ $C_1$- bis $C_4$-Alkyl,
$R^3$ Wasserstoff und
$R^4$ und $R^5$ die vorstehend angegebene Bedeutung haben. Von diesen sind solche Verbindungen bevorzugt, bei denen $R^1$ und $R^2$ für tert.-Butyl, $R^3$ für Wasserstoff und $R^4$ und $R^5$ unabhängig voneinander für $C_1$- bis $C_4$-Alkyl oder Benzyl stehen.

Aus coloristischen und anwendungstechnischen Gründen sind die Verbindungen der folgenden Formel besonders bevorzugt:

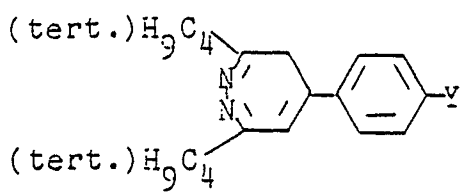 III,

worin Y für N,N-Dimethylamino, N,N-Diäthylamino, N,N-Di-n-butylamino, N,N-Dibenzylamino oder N-Äthyl-N-benzylamino steht.

In Research Disclosure 16629/1978 werden einige der genannten Diazepinverbindungen als farbbildende Komponente für photographische (lichtempfindliche) Schichten beschrieben.

Die Diazepine werden nach bekannten Verfahren durch Umsetzen von Pyryliumverbindungen mit Hydrazinhydrat in mit Wasser mischbaren Lösungsmitteln hergestellt.

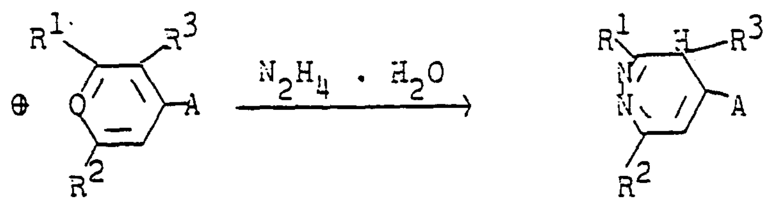


(IV) (I)

(Balaban, Tetrahedron 24, 5059 (1968); Buchardt, Acta chem. Scand. 23, 3125 (1969); Canad. J. Chem. 52, 2798 1974), Research Disclosure, Februar 1978, 16 629).

Als Ausgangsverbindungen kommen die Pyryliumsalze in Form ihrer Chloride, Perchlorate, Tetrafluoroborate, Tetrachloroferrate, Hydrogensulfate oder Trichlorozinkate in Betracht. Im einzelnen sind zu nennen:

2,6-Diphenyl-4-(4'-dimethylaminophenyl)-pyryliumsalz,
2,6-Di-tert.butyl-4-(4'-dimethylaminophenyl)-pyryliumsalz,
2,6-Di-tert.butyl-4-(4'-di-n-butylaminophenyl)-pyryliumsalz,
2,6-Diphenyl-4-(1'-methyl-2'-phenyl-3'-indolyl)-pyryliumsalz,
2,6-Diphenyl-4-(4'-dibenzylaminophenyl)-pyryliumsalz,
2,6-Di-tert.butyl-4-(4'-dibenzylaminophenyl)-pyryliumsalz,
2,6-Di-methyl-4-(4'-dimethylaminophenyl)-pyryliumsalz,
2,6-Di-tert.butyl-4-(4'-phenylmethylamino-1'-naphthyl)-pyryliumsalz,
2,6-Diphenyl-4-(4'-morpholinophenyl)-pyryliumsalz,
2,6-Di-p-methoxyphenyl-4-(4'-dimethylamino-phenyl)-pyryliumsalz,
2,6-Di-p-chlorphenyl-4-(4'-dimethylamino-phenyl)-pyryliumsalz.

Die Herstellung der erfindungsgemäß zu verwendenden Diazepine der Formel I wird in den folgenden Beispielen weiter erläutert. Die im folgenden genannten Teile und Prozentangaben beziehen sich auf das Gewicht.

Beispiel 1

a) 78 Teile 2,6-Diphenyl-4-(4'-dimethylamino-phenyl)-pyryliumchlorid und 60 Teile Hydrazinhydrat werden in 500 Teilen Methanol drei Stunden unter Rückfluß erhitzt. Das Reaktionsgemisch wird mit 1000 Teilen Wasser versetzt und mit 250 Teilen Toluol extrahiert. Die Toluolphase wird abgetrennt, über $Na_2SO_4$ getrocknet und auf ein Volumen von ca. 50 Teilen eingeengt. Durch Zugabe von 100 Teilen Äthanol werden 15 Teile 3,7-Diphenyl-5-(4'-dimethyl-aminophenyl)-4H-1,2-diazepin der Formel

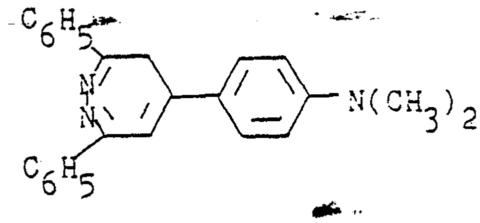

in Form von schwach gelb gefärbten Kristallen gefällt. Der Schmelzpunkt der Verbindung liegt bei 218 bis 220°C.

b) Eine Lösung dieser Verbindung wird nach dem in der DE-PS 21 19 933, Beispiel 1, beschriebenen Verfahren in Mikrokapseln eingeschlossen und auf Papier als Beschichtung aufgebracht. Man erhält beim Auflegen und Beschriften auf einer sauren Nehmerschicht, wobei die Kapseln zerstört werden und deren Inhalt mit der Nehmerschicht in Berührung gebracht wird, eine rote Durchschrift.

Beispiel 2

102 Teile 2,6-Di-tert.butyl-4-(4'-dimethylaminophenyl)-pyryliumtetrachloroferrat und 60 Teile Hydrazinhydrat werden in 500 Teilen Methanol 4 Stunden unter Rückfluß erhitzt. Das Reaktionsgemisch wird analog den Angaben in Beispiel 1 aufgearbeitet. Man erhält 47 Teile 3,7-Di-tert.-butyl-5-(4'-dimethylaminophenyl)-4H-1,2-diazepin der Formel

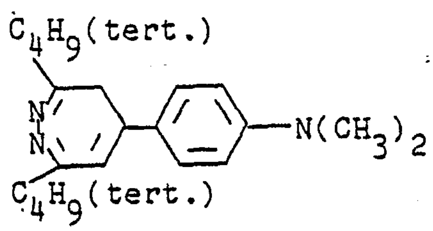

In Form farbloser Kristalle mit einem Schmelzpunkt von 174 bis 176°C.

In Kontakt mit sauer reagierenden Substanzen erhält man eine Orangefärbung.

Beispiel 3

45 Teile 2,6-Di-tert.butyl-4-(4'-di-n-butylaminophenyl)-pyryliumtetrachloroferrat werden entsprechend den Angaben in Beispiel 1 mit 20 Teilen Hydrazinhydrat in 150 Teilen Methanol umgesetzt. Man erhält die Verbindung 3,7-Di-tert.-butyl-5-(4'-di-n-butylaminophenyl)-4H-1,2-diazepin der Formel

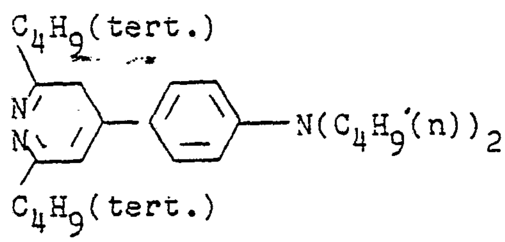

mit einem Schmelzpunkt von 68 bis 70°C. Ausbeute: 15 Teile.

Die Verbindung ergibt mit Elektronenakzeptorsubstanzen eine Orangefärbung.

Beispiel 4

70 Teile 2,6-Diphenyl-4-(4'-dibenzyl-aminophenyl)-pyrylium-tetrachloroferrat werden entsprechend den Angaben in Beispiel 1 mit 30 Teilen Hydrazinhydrat umgesetzt. Man erhält 32 Teile 3,7-Diphenyl-5-(4'-dibenzylaminophenyl)-4H-1,2-diazepin der Formel

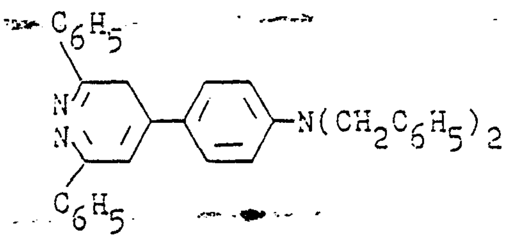

Die Verbindung schmilzt bei 158 bis 159° und ergibt mit sauer reagierenden Substanzen eine Orangefärbung.

Beispiel 5

Setzt man 27 Teile 2,6-Di-tert.butyl-4-(4'-dibenzylaminophenyl)-pyrylium-tetrachloroferrat analog Beispiel 1 mit 16 Teilen Hydrazinhydrat um, so isoliert man 10 Teile 3,7-Di-tert.butyl-5-(4'-dibenzylaminophenyl)-4H-1,2-diazepin der Formel

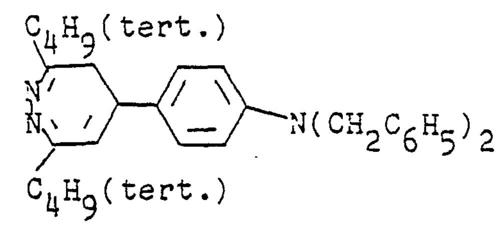

mit einem Schmelzpunkt von 158 bis 160°C.

In Kontakt mit sauer reagierenden Substanzen erhält man eine Orangefärbung.

Analog den Angaben in Beispiel 1 werden die Farbbildner der Formel

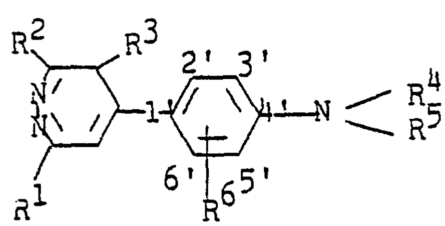

BAD ORIGINAL

erhalten. Die Bedeutung von $R^1$, $R^2$, $R^3$, $R^4$, $R^5$ und $R^6$ sowie der Farbton auf mit aktivem Clay beschichtetem Papier ist in der folgenden Tabelle angegeben:

| Bsp. | $R^1$ | $R^2$ | $R^3$ | $R^4$ | $R^5$ | $R^6$ | Farbton |
|---|---|---|---|---|---|---|---|
| 6 | $-CH_3$ | $-CH_3$ | -H | $-CH_3$ | $-CH_3$ | -H | orange |
| 7 | tert-$C_4H_9$ | tert-$C_4H_9$ | -H | $-C_2H_5$ | $-C_2H_5$ | 2'-$OC_2H_5$ | orange |
| 8 | -H | -H | -H | $-CH_3$ | $-CH_3$ | -H | gelborange |
| 9 | $-C_6H_5$ | $-C_6H_5$ | -H | $-CH_3$ | $-CH_2C_6H_5$ | -H | rot |
| 10 | tert-$C_4H_9$ | tert-$C_4H_9$ | -H | $-C_2H_5$ | $-CH_2C_6H_5$ | -H | orange |
| 11 | " | " | -H | $-(CH_2)_2O(CH_2)_2-$ | | -H | gelborange |
| 12 | " | " | -H | $-(CH_2)_4-$ | | -H | orange |
| 13 | " | " | -H | $-(CH_2)_5-$ | | -H | orange |
| 14 | " | " | -H | $-(CH_2)_2-\underset{CH_3}{N}-(CH_2)_2-$ | | -H | gelborange |
| 15 | " | " | -H | 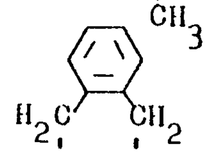 | | -H | gelborange |
| 16 | $-C_6H_5$ | $-(CH_2)_3-$ | | $-CH_3$ | $-CH_3$ | -H | gelborange |
| 17 | $-C_6H_5$ | $-(CH_2)_4-$ | | $-CH_3$ | $-CH_3$ | -H | gelborange |
| 18 | tert-$C_4H_9$ | tert-$C_4H_9$ | -H | $-C_6H_{13}$ | $-C_6H_{13}$ | -H | orange |
| 19 | tert-$C_4H_9$ | tert-$C_4H_9$ | -H | $-C_{12}H_{25}$ | $-C_{12}H_{25}$ | -H | orange |
| 20 | p-$C_6H_4OCH_3$ | p-$C_6H_4OCH_3$ | -H | $-C_2H_4CN$ | $-C_2H_4CN$ | -H | rot |
| 21 | p-$C_6H_4Cl$ | p-$C_6H_4Cl$ | -H | $-C_2H_4CN$ | $-CH_2C_6H_5$ | -H | rot |
| 22 | tert-$C_4H_9$ | tert-$C_4H_9$ | -H | $-CH_2C_6H_5$ | $-p-C_6H_4-CH_3$ | -H | orange |

| Bsp. | $R^1$ | $R^2$ | $R^3$ | $R^4$ | $R^5$ | $R^6$ | Farbton |
|---|---|---|---|---|---|---|---|
| 23 | tert-$C_4H_9$ | tert-$C_4H_9$ | -H | $-C_2H_4C_6H_5$ | $-C_2H_4C_6H_5$ | -H | orange |
| 24 | $-C_6H_5$ | $-C_6H_5$ | -H | $-C_2H_5$ | $-CH_2C_6H_5$ | 2'-$CH_3$ | rot |
| 25 | $-C_6H_5$ | $-C_6H_5$ | -H | -H | $-C_2H_5$ | 3'-$CH_3$ | rot |
| 26 | $-C_6H_5$ | $-C_6H_5$ | -H | -H | $-CH_3$ | -H | rot |
| 27 | $-C_6H_5$ | $-C_6H_5$ | -H | -H | -H | -H | rot |
| 28 | tert-$C_4H_9$ | tert-$C_4H_9$ | -H | $-CH_3$ | $-C_2H_4CN$ | -H | gelborange |
| 29 | $-C_6H_5$ | $-C_6H_5$ | -H | $-C_2H_5$ | $-C_2H_5$ | -H | rot |
| 30 | tert-$C_4H_9$ | tert-$C_4H_9$ | -H | $-C_6H_{12}Cl$ | $-C_4H_9$ | -H | orange |
| 31 | -H | -H | -H | $-CH_3$ | $-CH_2C_6H_5$ | -H | gelborange |
| 32 | tert-$C_4H_9$ | tert-$C_4H_9$ | -H | $-CH_3$ | $-CH_3$ | 2'-Cl | orange |
| 33 | tert-$C_4H_9$ | tert-$C_4H_9$ | -H | $-CH_3$ | $-CH_3$ | 2'-Br | orange |
| 34 | tert-$C_4H_9$ | tert-$C_4H_9$ | -H | $-CH_3$ | p-$C_6H_4OCH_3$ | -H | orange |

Beispiel 35

53 Teile 2,6-Diphenyl-4-(4'-phenyl-methylamino-1'-naphthyl)-pyryliumtetrachloroferrat werden entsprechend den Angaben in Beispiel 1 mit 40 Teilen Hydrazinhydrat umgesetzt. Die Ausbeute an 3,7-Diphenyl-5-(4'-phenyl-methylamino-1'-naphthyl)-4H-1,2-diazepin beträgt 9 Teile.

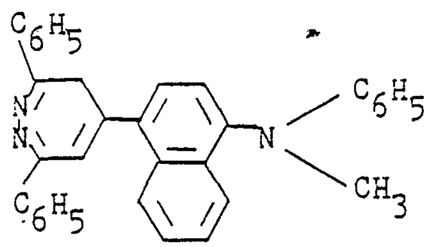

Die Verbindung schmilzt bei 125 - 127°C.

In Kontakt mit elektronenanziehenden Materialien erhält man eine Rotfärbung.

Analog den Angaben in Beispiel 35 werden Farbbildner der Formel

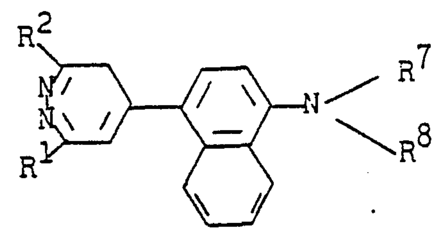

erhalten. Die Bedeutung von $R^1$, $R^2$, $R^7$ und $R^8$ sowie der Farbton auf mit aktivem Clay beschichtetem Papier ist in der folgenden Tabelle angegeben.

| Bsp. | $R^1$ | $R^2$ | $R^7$ | $R^3$ | Farbton |
|---|---|---|---|---|---|
| 36 | tert-$C_4H_9$ | tert-$C_4H_9$ | $-CH_3$ | $-C_6H_5$ | orange |
| 37 | $-CH_3$ | $-CH_3$ | $-CH_3$ | $-C_6H_5$ | orange |
| 38 | tert-$C_4H_9$ | tert-$C_4H_9$ | $-H$ | $-C_2H_5$ | orange |
| 39 | " | " | $-H$ | $-C_6H_5$ | orange |
| 40 | " | " | $-H$ | 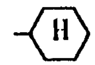 | orange |
| 41 | " | " | $-H$ | p-$C_6H_4CH_3$ | orange |
| 42 | " | " | $-H$ | p-$C_6H_4$-$OC_2H_5$ | orange |

Beispiel 43

70 Teile 2,6-Diphenyl-4-(1'-methyl-2'-phenyl-3'-indolyl)-pyryliumtetrachloroferrat und 30 Teile Hydrazinhydrat werden analog Beispiel 1 umgesetzt. Man erhält 27 Teile der Verbindung 3,7-Diphenyl-5-(1'-methyl-2'-phenyl-3'-indolyl)-4H-1,2-diazepin der Formel

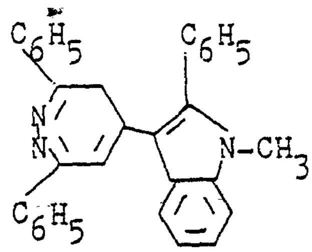

mit einem Schmelzpunkt von 202 bis 204°C.

In Kontakt mit sauer reagierenden Substanzen erhält man eine Gelbfärbung.

Analog den Angaben in Beispiel 43 werden Farbbildner der Formel

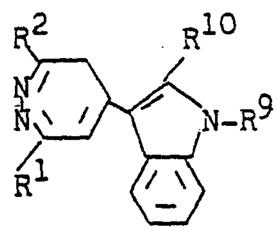

erhalten. Die Bedeutung der Substituenten $R^1$, $R^2$, $R^9$ und $R^{10}$, sowie der auf mit aktivem Clay beschichtetem Papier erzeugte Farbton sind in der folgenden Tabelle angegeben:

| Bsp. | $R^1$ | $R^2$ | $R^9$ | $R^{10}$ | Farbton |
|---|---|---|---|---|---|
| 44 | $-C_6H_5$ | $-C_6H_5$ | -H | $-C_6H_5$ | gelb |
| 45 | tert-$C_4H_9$ | tert-$C_4H_9$ | -H | $-CH_3$ | gelb |
| 46 | " | " | $-CH_3$ | $-CH_3$ | gelb |
| 47 | " | " | $-CH_3$ | $-C_8H_{17}$ | gelb |
| 48 | " | " | $-CH_3$ | p-$C_6H_4-CH_3$ | gelb |
| 49 | " | " | $-CH_3$ | p-$C_6H_4-OCH_3$ | gelb |
| 50 | $-C_6H_5$ | $-C_6H_5$ | $-CH_2C_6H_5$ | $-C_6H_5$ | gelb |
| 51 | $-C_6H_5$ | $-C_6H_5$ | $-C_2H_4C_6H_5$ | $-C_6H_5$ | gelb |

BASF Aktiengesellschaft O. Z. 0050/033534

## Druckempfindliches Aufzeichnungsmaterial

Die Erfindung betrifft druckempfindliches Aufzeichnungsmaterial, das als Farbbildner Verbindungen der allgemeinen Formel

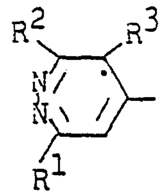 (I)

enthält, in der

$R^1$ und $R^2$ unabhängig voneinander Wasserstoff, $C_1$- bis $C_4$-Alkyl, Phenyl oder durch $C_1$- bis $C_4$-Alkyl, Methoxy, Äthoxy, Chlor oder Brom substituiertes Phenyl,

$R^3$ Wasserstoff oder zusammen mit $R^2$ einen gesättigten carbocyclischen Fünf- oder Sechsring und

A einen Rest der Formel

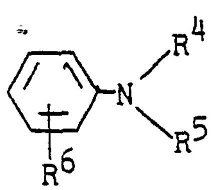 , 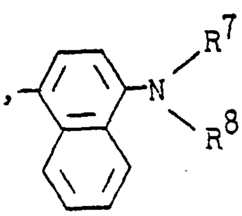 oder 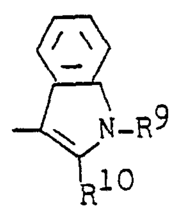

(IIa) (IIb) (IIc)

bedeuten, worin

$R^4$ und $R^5$ unabhängig voneinander für Wasserstoff, gegebenenfalls durch Chlor substituiertes $C_1$- bis $C_{12}$-Alkyl, Cyanäthyl, $C_7$- bis $C_{10}$-Phenalkyl oder die Gruppe

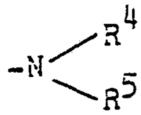 für Pyrrolidinyl, Piperidinyl, Morpholinyl, N'-$C_1$- bis $C_4$-Alkyl-piperazinyl oder Isoindolinyl, oder

$R^4$ für Wasserstoff, $C_1$- bis $C_4$-Alkyl, 2-Cyanäthyl oder Benzyl und

$R^5$ für Phenyl oder durch Halogen, $C_1$- bis $C_4$-Alkyl oder $C_1$- bis $C_4$-Alkoxy substituiertes Phenyl,

$R^6$ für Wasserstoff, $C_1$- bis $C_4$-Alkyl, $C_1$- bis $C_4$-Alkoxy, Chlor oder Brom,

$R^7$ für Wasserstoff oder $C_1$- bis $C_4$-Alkyl,

$R^8$ für $C_1$- bis $C_4$-Alkyl, Phenyl, durch $C_1$- bis $C_4$-Alkyl, $C_1$- bis $C_4$-Alkoxy substituiertes Phenyl oder Cyclohexyl,

$R^9$ für Wasserstoff, $C_1$- bis $C_{12}$-Alkyl oder $C_7$- bis $C_{10}$-Phenalkyl und

$R^{10}$ für Wasserstoff, $C_1$- bis $C_{10}$-Alkyl, Phenyl oder durch $C_1$- bis $C_4$-Alkyl oder $C_1$- bis $C_4$-Alkoxy substituiertes Phenyl stehen.

Als Substituenten kommen für $R^1$ und $R^2$ z.B. im einzelnen in Betracht: Methyl, Äthyl, Propyl, Butyl, Isobutyl, tert.-Butyl, Phenyl, Methoxyphenyl, Chlorphenyl, Bromphenyl, Äthoxyphenyl, Methylphenyl, Äthylphenyl. Vorzugsweise stehen $R^1$ und $R^2$ für $C_1$- bis $C_4$-Alkyl.

Für $R^4$ und $R^5$ sind außer Wasserstoff und Cyanäthyl z.B. im einzelnen zu nennen: gegebenenfalls durch Chlor substitu-

4. Druckempfindliches Aufzeichnungsmaterial gemäß Anspruch 2, dadurch gekennzeichnet, daß $R^1$ und $R^2$ für tert.-Butyl, $R^3$ für Wasserstoff und

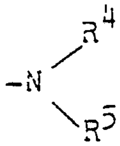

für N,N-Dimethylamino, N,N-Diäthylamino, N,N-Di-n-butylamino, N,N-Dibenzylamino oder N-Äthyl-N-benzylamino stehen.